(19) Europäisches Patentamt
European Patent Office
Office européen des brevets

(11) EP 0 645 143 B1

(12) **EUROPEAN PATENT SPECIFICATION**

(45) Date of publication and mention of the grant of the patent:
**31.05.2000 Bulletin 2000/22**

(51) Int. Cl.[7]: **A61K 35/80**

(21) Application number: **94114879.3**

(22) Date of filing: **21.09.1994**

(54) **Antiulcer agent and adhesion inhibitor for Helicobacter pylori**

Antiulcerogenes Mittel und Adhäsionsinhibitor für Helicobacter pylori

Agent anti-ulcéreux et inhibiteur d'adhésion de Hélicobacter pylori

(84) Designated Contracting States:
**AT BE CH DE DK ES FR GB GR IE IT LI LU MC NL PT SE**

(30) Priority: **24.09.1993 JP 25888393**
**28.04.1994 JP 11194694**

(43) Date of publication of application:
**29.03.1995 Bulletin 1995/13**

(73) Proprietor:
**Kabushiki Kaisha Yakult Honsha**
**Minato-ku, Tokyo 105-0021 (JP)**

(72) Inventors:
- **Kimura, Itsuko**
  **1-chome, Minato-ku, Tokyo (JP)**
- **Shibata, Hideyuki**
  **1-chome, Minato-ku, Tokyo (JP)**
- **Nagaoka, Masato**
  **1-chome, Minato-ku, Tokyo (JP)**
- **Hashimoto, Shusuke**
  **1-chome, Minato-ku, Tokyo (JP)**
- **Sawada, Haruji**
  **1-chome, Minato-ku, Tokyo (JP)**
- **Yokokura, Teruo**
  **1-chome, Minato-ku, Tokyo (JP)**

(74) Representative:
**Kraus, Walter, Dr. et al**
**Patentanwälte Kraus, Weisert & Partner**
**Thomas-Wimmer-Ring 15**
**80539 München (DE)**

(56) References cited:
**WO-A-88/05301**

- **PATENT ABSTRACTS OF JAPAN, unexamined applications, C field, vol. 7, no. 11, January 18, 1983 THE PATENT OFFICE JAPANESE GOVERNMENT page 135 C 145; & JP-A-57 169 421 (YOSHIO SAKAGAMI)**
- **PATENT ABSTRACTS OF JAPAN, unexamined applications, C field, vol. 7, no. 11, January 18, 1983 THE PATENT OFFICE JAPANESE GOVERNMENT page 135 C 145; & JP-A-57 169 422 (YOSHIO SAKAGAMI)**
- **PATENT ABSTRACTS OF JAPAN, unexamined applications, C field, vol. 14, no. 109, February 28, 1990 THE PATENT OFFICE JAPANESE GOVERNMENT page 117 C 695; & JP-A-01 313 434 (ASAHI)**
- **PATENT ABSTRACTS OF JAPAN, unexamined applications, C field, vol. 13, no. 293, July 6, 1989 THE PATENT OFFICE JAPANESE GOVERNMENT page 85 C 615; & JP-A-01 87 601 (YOSHIO ITAYA)**
- **CHEMICAL ABSTRACTS, vol. 102, no. 25, June 24, 1985, Columbus, Ohio, USA GRABEL, L.B. "Isolation of a putative cell adhesion mediating lectin from teratocarcinoma stem cells and its possible role in differentiation" page 474, column 1, abstract-no. 219 477u; & Cell Differ. 1984, 15(2-4), 121-4**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 645 143 B1

## Description

FIELD OF THE INVENTION

This invention relates to the use of a drug useful in the preparation of a medicament for preventing and treating ulcers and tumors on the gastric mucosa, and other disorders.

BACKGROUND OF THE INVENTION

As antiulcer agents for treating ulcers on the gastric mucosa, $H_2$ blockers and proton pump inhibitors which aim at suppressing gastric acid secretion, have been used. However, gastric ulcers frequently recur after discontinuation of the administration of these secretion-suppressing drugs.

In recent years, a certain bacterium *Helicobacter pylori* has been identified as participating in the occurrence of ulcers on the gastric mucosa. This bacterium is believed to adhere to the gastric mucosa, where it produces ammonia which induces gastritis and, in its turn, gastric ulcer or cancer. See Anti-Infectious Agent and Diseases, 1993, pp. 294-309.

Because this bacterium adheres to the gastric submucous tissue, extermining the bacterium by administering drugs such as antibiotics is very difficult. Although attempts have been made to exterminate *Helicobacter pylori* by the combined use of several antibiotics with proton pump inhibitors, such treatments must administer a large amounts of antibiotics over a long time period. In such treatments, various problems accompanying the appearance of an antibiotic-tolerant strain undesirably might be induced.

*Helicobacter pylori* recognizes cholesterol, glycolipids, sulfated glycolipids, laminin, fucosylated sugar chains and sialic acid on the gastric mucosa and thus adheres to the gastric mucosa (Moran, A.P. et al., Journal of Applied Bacteriology 1993, 75, 184-189; Lingwood, C.A. et al., Infection and Immunity 1993, 61, 2474-2478). Therefore, attempts have been made to prevent gastric mucosa inflammation due to *Helicobacter pylori* by interfering with the process of the recognition of these compounds by *Helicobacter pylori* and the adhesion of the bacterium to the gastric mucosa (Huesca, M. et al., Zbl. Bakt. 1993, 280, 244-252). Examples of substances which have been reported as inhibitors for the adhesion of *Helicobacter pylori* include fetuin (which inhibits the adhesion via sialic acid) and secretory IgA (which inhibits the adhesion via a fucosylated sugar chain). However, these substances have not been put into practical use as adhesion inhibitors for *Helicobacter pylori*, since they cannot he supplied on a mass scale.

On the other hand, the present inventors previously have found that the cells of a certain bacterium belonging to the genus *Bifidobacterium* or lactic acid bacteria and polysaccharides extracted therefrom are effective not only in preventing gastric ulcers, but also in promoting the healing thereof, and thus are usable as an antiulcer agent (JPA-4-5236; the term "JPA" as used herein means an "unexamined published Japanese patent application"). The present inventors further discovered that rhamnose rich polysaccharides, rhamnan and rhamnose oligosaccharides originating in certain marine algae exert similar effects (JPA-5-61447). However, the antiulcer mechanism exhibited by the abovementiond polysaccharides has not been clarified previously.

WO 88/05301 refers to a method of anti-metastatic and/or anti-inflammatory treatment of an animal or human patient which comprises the administration to the patient of an effective amount of at least one sulphated polysaccharide which blocks or inhibits endoglycosidase, particularly heparanase activity. Suitable sulphated polysaccharides include heparin and modified heparin, fucoidan, pentosan sulphate, dextran sulphate and carrageenan lambda. This document does not mention that fucoidan is useful as an antiulcer agent for the manufacture of a medicament for preventing and treating ulcers and tumors of the gastric mucosa caused by *Helicobacter pylori.*

DE 21 62 343 refers to sulphated polysaccharides such as carragenine, chondroitin sulphate, polyhydromannuron sulphate and amylopectine sulphate. These sulphated polysaccharides are used to inhibit the activity of pepsine. The document further discloses levane sulphate that can be used as a pharmaceutical having antipeptic activity. The document does not mention fucoidan. Furthermore, DE 21 62 343 does not mention *Helicobacter pylori* and does not point to the fact that the adhesion of *Helicobacter pylori* can be inhibited by the use of fucoidan.

SUMMARY OF THE INVENTION

It is an object of the present invention to provide the use of an antiulcer agent for the manufacture of a medicament effective in treating and preventing ulcers.

It is another object of the present invention to provide the use of a drug for the manufacture of a medicament which inhibits the adhesion of *Helicobacter pylori* on the gastric mucosa, and thus enables the prevention and treatment of gastritis, gastric ulcers, gastric cancer, and other disorders caused by said bacterium.

Both the antiulcer agent and the adhesion inhibitor for *Helicobacter pylori* as used according to the present invention (hereinafter called "the drug as used according to the present invention" and intended to include both of these

agents) comprise fucoidan (i.e., polysaccharides mainly consisting of fucose) as an active ingredient.

The invention is summarized in the claims.

DETAILED DESCRIPTION OF THE INVENTION

Fucoidan been marketed in the form of reagents and purified preparations. Using these commercially available products as the starting material to prepare the drug as used according to the present invention is most convenient. Alternately, fucoidan can be extracted easily from red or brown algae rich in fucoidan, with hot water or an aqueous solution of a diluted acid. Such an extract and a purified product are also usable according to the present invention. Fucoidan extracted from red or brown algae usually has a molecular weight of about 100,000 and comprises about 50 to 60 % fucose by weight, with a small amount of uronic acid. Some of the sugars constituting the fucoidan have been sulfated.

Examples of the marine algae which are rich in fucoidan and advantageously used as a starting material for the drug as used according to the present invention include red algae such as *Phaeophyceae Chordariales nemacystus* and brown algae such as *Padina arborescens*, *Undaria pinnatifida*, *Fucus evanescens*, *Himanthulia*, *Bifurcaria* and *Fucus vesiculosus.*

Typical examples of the method for extracting fucoidan from fucoidan-containing algae are as follows.

(1) Hot water extraction

A fucoidan-containing alga is suspended in about two to three times as much (on a wet basis) water and heated to about 100 °C for about 10 minutes to 1 hour. After removing the precipitate by centrifugation, calcium chloride or barium acetate is added to the supernatant to give a concentration of 0.5 to 1.0 g/l and alginic acid thus precipitated is removed. Then low molecular weight substances are removed by three times dialysis against 10 times as much distilled water, or ultrafiltration and the residue is freeze-dried to give fucoidan.

(2) Acid extraction

A fucoidan-containing alga is suspended in about three times as much (on a wet basis) aqueous solution of acetic acid to adjust pH 4 or dilute hydrochloric acid (pH 2.0) to give a concentration of 0.01 mol and extracted, at about room temperature to 100 °C. After removing the precipitate by centrifugation, the supernatant is neutralized by adding a solution of caustic soda. Then a solution of calcium chloride is added thereto to give a concentration of 0.5 to 1.0 g/l and alginic acid thus precipitated is removed. Next, low molecular weight substances are removed by ultrafiltration and dialysis and the residue is freeze-dried to give fucoidan.

The ultrafiltration is conducted using an ultrafiltration membrane with a fractional molecular weight of 5,000 to concentrate the resulting supernatant to one tenth. The concentrate thus obtained is three times dialyzed against ten times as much distilled water in dialysis tube with a fractional molecular weight of 6,000 to 8,000 (dialysis time: 20 hr).

The extract from either extraction method may be further purified by, for example, gel filtration or ion exchange chromatography to elevate the content of fucoidan.

The high molecular weight fucoidan extracted from marine algae from either extraction method may be degraded to an average molecular weight of about 10,000 by, for example, Smith degradation comprising treating with periodic acid. Seikagaku Jikken Koza, 4 "Toshitsu no Kagaku" the last volume (Biochemical Experiment Lecture 4 "Chemistry of Saccharides" the last volume, Tokyo Kagaku Dojin. See also Example 3 hereinafter). The degraded fucoidan represented by the following formula may be used according to the present invention:

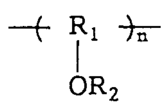

wherein, $R_1$ is fucose, $R_2$ is H or $SO_3^-$ and n is an integer. Degradation advantageously lowers the viscosity of the solution and thus makes handling the solution easy without causing decreased efficacy.

Fucoidan is at least comparable and in some cases superior in antiulcer effect to polysaccharides extracted from bifidobacteria and marine algae.

The antiulcer agent and adhesion inhibitor for *Helicobacter pylori* as used according to the present invention are orally administered. Fucoidan enters the digestive tract and reaches the affected part where it exerts an ulcer-treating effect.

Fucoidan also prevents gastric mucosa inflammation, which advances into gastric ulcer or cancer, by inhibiting the adhesion of *Helicobacter pylori* to the gastric mucosa, (See Test Example 4 herein).

The drug as used according to the present invention comprises fucoidan, i.e., polysaccharides originating in edible algae, and thus is very safe. Accordingly, the dosage form and dose can be selected arbitrarily. In general, the drug as used according to the present invention may be blended with pharmaceutically acceptable liquids or solid carriers optionally together with, for example, solvents, dispersing agents, emulsifiers, buffers, stabilizers, fillers, binders, disintegrants and lubricants, and may be formulated into, for example, tablets, granules, dusts, powders and capsules.

The dose of the drug as used according to the present invention appropriately ranges from about 1 to 500 mg/kg/day, preferably from 5 to 50 mg/kg/day, for most adults.

This drug as used according to the present invention may be orally administered. Alternately, it may be added to food or drink for daily intake.

EXAMPLE 1

*Phaeophyceae Chordariales nemacystus* was washed with distilled water and 3 ml of water was added to every 1 g of the alga (on a wet basis) of water was added thereto. Then the solution was heated to 100 °C for 1 hour to extract components soluble in hot water. The extract thus obtained was filtered through a Nylon cloth filter. The filtrate was centrifuged at 9,000 rpm for 60 minutes. The resulting supernatant was concentrated, using an ultrafiltration membrane with a fractional molecular weight of 30,000. Ethanol was added to the concentrate to give a concentration of 30 to 50 vol%. The mixture then was centrifuged to eliminate the insoluble matters.

The supernatant thus obtained was dialyzed against distilled water and freeze-dried. Thus fucoidan (purity: 90 % or above) was obtained. Yield: 0.5 % by weight (based on wet algae).

The purity was measured by NMR, the amount of neutral saccharides determined by phenol-sulfuric acid method and the amount of protein determined by Micro BCA method.

The components of the fucoidan thus obtained were analyzed by gas chromatography. The fucoidan comprised 70 to 80 % fucose as the main component and 0 to 5 % uronic acid. From gel filtration using Sephacryl S-200, the molecular weight of the fucoidan was estimated as about 100,000. Data of $^{13}C$-NMR indicated that these polysaccharides comprised 70 to 80 % 6-deoxysaccharide polymers as the main component and 0 to 5 % uronic acid.

EXAMPLE 2

The procedure of the above Example 1 was repeated, except that the fucoidan extract was extracted from *Phaeophyceae Chordariales nemacystus* with 0.01 N hydrochloric acid at 100 °C for 30 minutes to give fucoidan (yield: 1.5 % by weight based on wet algae).

The fucoidan thus obtained had a purity of 90 % or above and contained 3377 nmol/mg of fucose and 1442 nmol/mg of sulfate group.

EXAMPLE 3

7 g of the fucoidan obtained in the above Example 1 was dissolved in 500 ml of a 0.2 M solution of periodic acid (in 10 mM acetate buffer, pH 5.0) and allowed to stand in the dark for 3 days. Then 1 ml of ethylene glycol was added to decompose the excessive periodic acid. The reaction mixture was adjusted to pH 8.0 by adding 1M borate buffer to give a final concentration of 0.4 M and then reduced by adding 4 g of sodium borohydride. After the completion of the reaction, the mixture was neutralized by adding glacial acetic acid and concentrated and desalted by ultrafiltration. Next, the pH value of the mixture was adjusted to 2.0 by adding hydrochloric acid and deacetalyzation was performed. The reaction mixture was dialyzed using a dialysis membrane of a fractional molecular weight of 1,000. The resulting inner liquor was freeze-dried. Thus degraded fucoidan (average molecular weight: about 10,000, yield: 3.44 g) was obtained.

TEST EXAMPLE 1

The fucoidan prepared in Examples 1 and 2 and commercially available fucoidan were tested for antiulcer activity on acetic acid-induced ulceration.

Laparotomy was performed on ten 8-week-old SD rats (body weight: 250-300 g), under anesthesia with Nembutal. The stomach was exposed, and 0.03 mℓ of 20% acetic acid was injected in the submucous coat of the corpus ventriculi to induce ulceration. The test substance was orally given to the animals at a daily dose shown in Tables 1-3 below from the 2nd to 9th days from the operation. During the testing period, the animals were fed food and water, ad lib. On the 10th day, the stomachs were excised, and the area of the ulcerated part (longer diameter x shorter diameter) was measured as an ulceration index. "Percent healing" was calculated from the ulceration index according to the

following equation:

Percent healing (%) = (1 - Ulceration index of test group/Ulceration index of control group) x 100

The results obtained are shown in Tables 1-3. The ulceration indices in the Tables are expressed as mean (of 10 animals in each group) ± standard deviation.

TABLE 1

| Antiulcer Effect of Fucoidan of Example 1 | | |
|---|---|---|
| Dose (mg/rat) | Ulceration Index | Percent Healing (%) |
| 0 (control) | 11.0±3.2 | - |
| 1.5 × 8 | 8.7±3.4 | 21.1 |
| 3.0 × 8 | 5.6±2.9* | 49.1 |
| 6.0 × 8 | 5.9±1.6* | 45.9 |

Note:
* Significant at an error of 5% or less (hereinafter the same)

TABLE 2

| Antiulcer Effect of Fucoidan of Example 2 | | |
|---|---|---|
| Dose (mg/rat) | Ulceration Index | Percent Healing (%) |
| 0 (control) | 12.8±3.9 | - |
| 1.5 × 8 | 7.6±3.6* | 40.4 |
| 3.0 × 8 | 9.2±5.3 | 28.4 |
| 6.0 × 8 | 8.7±4.0* | 32.0 |

Note:
* Significant at an error of 5% or less (hereinafter the same)

TABLE 3

| Antiulcer Effect of Fucoidan (derived from Fucus vesiculosus, manufactured by SIGMA CHEMICAL COMPANY) | | |
|---|---|---|
| Dose (mg/rat) | Ulceration Index | Percent Healing (%) |
| 0 (control) | 11.7±3.1 | - |
| 1.5 × 8 | 8.7±1.9* | 26.1 |
| 3.0 × 8 | 7.3±3.7* | 38.4 |
| 6.0 × 8 | 6.3±2.2* | 46.4 |

Note:
* Significant at an error of 5% or less (hereinafter the same)

TEST EXAMPLE 2

*Helicobacter pylori* was fixed on a microplate. Next, biotin-labelled BSA (bovine serum albumin) bound to a fucose sugar chain, which was seemingly an adhesion factor of *Helicobacter pylori* was added onto the microplate. Thus the BSA adhered to *Helicobacter pylori* The adhesion was determined by a color development reaction.

The fucoidan obtained in above Example 2 and the degraded fucoidan obtained in above Example 3 were examined for activity in inhibiting the adhesion of *Helicobacter pylori* using the degree of the inhibition of the above-mentioned adhesion due to adding each fucoidan as an indicator. The determination was performed in the following manner.

The biotin-labeled and fucose sugar chain-binding BSA (0 to 0.04 µg/ml) were added to each well of the plate (Pyrica plate, a product commercially available from Biomerica Co.) on which *Helicobacter pylori* had been fixed and reacted at 37 °C for 1 hour. After washing away the unbound sugar chain-binding BSA with a buffer solution, a solution of streptoavidin (concentration: 0.05 mg/ml) was added to the wells in 100 µl portions and reacted at room temperature for 30 minutes. Thus streptoavidin selectively bound to the biotin of the sugar chain-binding BSA remaining in each well. After washing away the excessive streptoavidin, 100 µl of a peroxidase-labeled biotin solution (2.5 µg/mg) was added and reacted at room temperature for 30 minutes. Then the excessive enzyme-labeled biotin was washed away. Thus the enzyme-labeled biotin selectively bound to the complex of the sugar chain-binding BSA and streptoavidin. After adding 100 µl of a substrate solution (an ABTS peroxidase substrate system, a product commercially available from KPL) and reacting at room temperature for 10 minutes, the activity of the enzyme (peroxidase) was measured as the absorbance at a wavelength of 405 nm. The absorbance thus measured increased depending on the amount of the fucose-binding BSA added to the reaction system.

5 to 150 µg/ml of fucoidan was added to the reaction system containing the fucose-binding BSA and *Helicobacter pylori* as described above and the absorbance was measured in the same manner. Tables 4 and 5 show the results. The absorbance thus measured decreased with increased amount of fucoidan added to the reaction system, indicating fucoidan inhibited the adhesion of *Helicobacter pylori* to the sugar chain-binding BSA.

TABLE 4

| Inhibitory Activity of Fucoidan of Example 2 on Adhesion of *Helicobacter pylori* | |
|---|---|
| Conc. of fucoidan (µg/ml) | Absorbance (405 nm) |
| 0 | 1.219 |
| 1 | 0.901 |
| 2.5 | 0.430 |
| 5 | 0.297 |
| 7.5 | 0.132 |
| 10 | 0.092 |
| 100 | 0.000 |

TABLE 5

| Inhibitory Activity of Degraded Fucoidan of Example 3 on Adhesion of *Helicobacter pylori* | |
|---|---|
| Conc. of fucoidan (µg/ml) | Absorbance (405 nm) |
| 0 | 1.284 |
| 10 | 1.254 |

TABLE 5 (continued)

| Inhibitory Activity of Degraded Fucoidan of Example 3 on Adhesion of *Helicobacter pylori* | |
|---|---|
| Conc. of fucoidan (μg/ml) | Absorbance (405 nm) |
| 15 | 0.895 |
| 20 | 0.725 |
| 25 | 0.501 |
| 50 | 0.209 |
| 100 | 0.098 |
| 150 | 0.092 |

As discussed above, the present invention provides the use of a drug which promotes healing of ulcers and inhibits the adhesion of *Helicobacter pylori* and thus is effective in preventing and treating gastric mucosa inflammation which might advance into an ulcer or cancer. The drug as used according to the present invention is produced using edible marine algae such as *Phaeophyceae Chordariales nemacystus* as a starting material. Thus, the drug as used according to the present invention can be supplied on a mass scale at low cost. Further the drug as used according to the present invention advantageously is very safe.

**Claims**

**1.** Use of an antiulcer agent comprising fucoidan as an active ingredient for the manufacture of a medicament for preventing and treating ulcers and tumors of the gastric mucosa caused by *Helicobacter pylori.*

**2.** Use according to claim 1 wherein the antiulcer agent comprises an algae extract containing fucoidan as an active ingredient, wherein said algae is red algae or brown algae.

**3.** Use according to any of claims 1 to 2 wherein the antiulcer agent comprises a *Phaeophyceae Chordariales nemacystus* extract containing fucoidan as an active ingredient.

**4.** Use according to any of claims 1 to 3 wherein the fucoidan is degraded.

**5.** Use according to claim 1, wherein the agent comprises fucoidan in a therapeutically effective amount.

**6.** Use of an adhesion inhibitor for *Helicobacter pylori* comprising fucoidan as an active ingredient for the manufacture of a medicament for inhibiting the adhesion of *Helicobacter pylori* on the gastric mucosa.

**7.** Use according to claim 6 wherein the adhesion inhibitor comprises an algae extract containing fucoidan as an active ingredient, wherein said algae is red algae or brown algae.

**8.** Use according to any of claims 6 to 7 wherein the adhesion inhibitor comprises a *Phaeophyceae Chordariales nemacystus* extract containing fucoidan as an active ingredient.

**9.** Use according to claim 6, wherein the agent comprises fucoidan in a therapeutically effective amount.

**10.** Use of an adhesion inhibitor for Helicobacter *pylori* comprising degraded fucoidan as an active ingredient for the manufacture of a medicament for inhibiting the adhesion of *Helicobacter pylori* on the gastric mucosa.

**11.** Use according to claim 10 wherein the adhesion inhibitor for *Helicobacter pylori* comprises an algae extract containing degraded fucoidan as an active ingredient, wherein said algae is red algae or brown algae.

**12.** Use according to any of claims 10 to 11 wherein the adhesion inhibitor comprises a *Phaeophyceae Chordariales nemacystus* extract containing degraded fucoidan as an active ingredient.

## Patentansprüche

1. Verwendung eines anti-ulcerösen Mittels, umfassend Fucoidan als einen aktiven Bestandteil zur Herstellung eines Medikaments zur Prävention und zur Behandlung von Geschwüren und Tumoren der Magenschleimhaut, die durch *Helicobacter pylori* verursacht werden.

2. Verwendung nach Anspruch 1, wobei das anti-ulceröse Mittel einen Algenextrakt umfaßt, der Fucoidan als einen aktiven Bestandteil enthält, wobei die Alge eine Rotalge oder Braunalge ist.

3. Verwendung nach einem der Ansprüche 1 bis 2, wobei das anti-ulceröse Mittel einen *Phaeophyceae Chordariales nemacystus* Extrakt umfaßt, der Fucoidan als einen aktiven Bestandteil enthält.

4. Verwendung nach einem der Ansprüche 1 bis 3, wobei das Fucoidan abgebaut ist.

5. Verwendung nach Anspruch 1, wobei das Mittel Fucoidan in einer therapeutisch wirksamen Menge umfaßt.

6. Verwendung eines *Helicobacter pylori* Adhäsionsinhibitors, umfassend Fucoidan als einen aktiven Bestandteil zur Herstellung eines Medikaments zum Inhibieren der Adhäsion von *Helicobacter pylori* an die Magenschleimhaut.

7. Verwendung nach Anspruch 6, wobei der Adhäsionsinhibitor einen Algenextrakt umfaßt, welcher Fucoidan als einen aktiven Bestandteil enthält, wobei die Alge eine Rotalge oder Braunalge ist.

8. Verwendung nach einem der Ansprüche 6 bis 7, wobei der Adhäsionsinhibitor einen *Phaeophyceae Chordariales nemacystus* Extrakt umfaßt, der Fucoidan als einen aktiven Bestandteil enthält.

9. Verwendung nach Anspruch 6, wobei das Mittel Fucoidan in einer therapeutisch wirksamen Menge umfaßt.

10. Verwendung eines *Helicobacter pylori* Adhäsionsinhibitors, umfassend abgebautes Fucoidan als einen aktiven Bestandteil zur Herstellung eines Medikaments zum Inhibieren der Adhäsion von *Helicobacter pylori* an die Magenschleimhaut.

11. Verwendung nach Anspruch 10, wobei der *Helicobacter pylori* Adhäsionsinhibitor einen Algenextrakt umfaßt, der abgebautes Fucoidan als einen aktiven Bestandteil enthält, wobei die Alge eine Rotalge oder eine Braunalge ist.

12. Verwendung nach einem der Ansprüche 10 bis 11, wobei der Adhäsionsinhibitor einen *Phaeophyceae Chordariales nemacystus* Extrakt umfaßt, der abgebautes Fucoidan als einen aktiven Bestandteil enthält.

## Revendications

1. Utilisation d'un agent antiulcéreux comprenant du fucoïdane en tant qu'ingrédient actif pour la fabrication d'un médicament pour la prévention et le traitement des ulcères et des tumeurs de la muqueuse gastrique induits par *Helicobacter pylori.*

2. Utilisation selon la revendication 1, dans laquelle l'agent antiulcéreux comprend un extrait d'algue contenant du fucoïdane en tant qu'ingrédient actif, ladite algue étant une algue rouge ou une algue brune.

3. Utilisation selon une quelconque des revendications 1 ou 2, dans laquelle l'agent antiulcéreux comprend un extrait de *Phaeophyceae Chordariales nemacystus* contenant du fucoïdane en tant qu'ingrédient actif.

4. Utilisation selon une quelconque des revendications 1 à 3, dans laquelle le fucoïdane est dégradé.

5. Utilisation selon la revendication 1, dans laquelle l'agent comprend une quantité thérapeutiquement efficace de fucoïdane.

6. Utilisation d'un inhibiteur d'adhérence pour *Helicobacter pylori* comprenant du fucoïdane en tant qu'ingrédient actif pour la fabrication d'un médicament pour l'inhibition de l'adhérence d'*Helicobacter pylori* sur la muqueuse gastrique.

**7.** Utilisation selon la revendication 6, dans laquelle l'inhibiteur d'adhérence comprend un extrait d'algue contenant du fucoïdane en tant qu'ingrédient actif, ladite algue étant une algue rouge ou une algue brune.

**8.** Utilisation selon une quelconque des revendications 6 ou 7, dans laquelle l'inhibiteur d'adhérence comprend un extrait de *Phaeophyceae Chordariales nemacystus* contenant du fucoïdane en tant qu'ingrédient actif.

**9.** Utilisation selon la revendication 6, dans laquelle l'agent comprend une quantité thérapeutiquement efficace de fucoïdane.

**10.** Utilisation d'un inhibiteur d'adhérence pour *Helicobacter pylori* comprenant du fucoïdane dégradé en tant qu'ingrédient actif pour la fabrication d'un médicament pour l'inhibition de l'adhérence d'*Helicobacter pylori* sur la muqueuse gastrique.

**11.** Utilisation selon la revendication 10, dans laquelle l'inhibiteur d'adhérence pour *Helicobacter pylori* comprend un extrait d'algue contenant du fucoïdane dégradé en tant qu'ingrédient actif, ladite algue étant une algue rouge ou une algue brune.

**12.** Utilisation selon une quelconque des revendications 10 ou 11, dans laquelle l'inhibiteur d' adhérence comprend un extrait de *Phaeophyceae Chordariales nemacystus* contenant du fucoïdane dégradé en tant qu'ingrédient actif.